# EUROPEAN PATENT SPECIFICATION

(11) **EP 3 564 665 B1**
(45) Date of publication and mention of the grant of the patent: **03.05.2023**
(21) Application number: 17887687.6
(22) Date of filing: 29.12.2017
(51) Int. Cl.: G01N 33/48, G01N 33/574, C12Q 1/6886, C40B 30/04, C40B 40/08

(54) **EX-VIVO METHOD FOR THE PROGNOSIS OF METASTASIS IN PROSTATE CANCER**
EX-VIVO-VERFAHREN ZUR PROGNOSE VON METASTASIERUNG BEI PROSTATAKREBS
MÉTHODE EX VIVO DE PRONOSTIC DE MÉTASTASES DU CANCER DE LA PROSTATE

(30) Priority: 30.12.2016 CL 20163434
(43) Date of publication of application: 06.11.2019
(73) Proprietor: Pontificia Universidad Católica De Chile, 8331150 Santiago (CL); Cerda Infante, Javier, Santiago 8331150 (CL)
(72) Inventor: CERDA INFANTE, Javier, Santiago 8331150 (CL); MONTECINOS ACUÑA, Viviana, Santiago 8331150 (CL)
(74) Representative: Elzaburu S.L.P.
(86) International application number: PCT/CL2017/050095
(87) International publication number: WO 2018/119544

(56) References cited:
- EP-A1- 2 762 574
- WO-A2-2006/135886
- WO-A2-2012/129408
- WO-A2-2014/052930
- CA-A1- 2 680 692
- CA-A1- 2 745 961
- US-A1- 2011 053 792
- US-A1- 2013 085 080
- US-A1- 2016 138 113
- ROBINSON MARK D ET AL: "A comparison of Affymetrix gene expression arrays", BMC BIOINFORMATICS, BIOMED CENTRAL, LONDON, GB, vol. 8, no. 1, 15 November 2007 (2007-11-15), page 449, XP021031592, ISSN: 1471-2105
- QINWEN WANG ET AL: "Stromal cell-derived factor-1 and vascular endothelial growth factor as biomarkers for lymph node metastasis and poor cancer-specific survival in prostate cancer patients after radical prostatectomy", UROLOGIC ONCOLOGY: SEMINARS AND ORIGINAL INVESTIGATIONS, vol. 31, no. 3, 1 April 2013 (2013-04-01), pages 312-317, XP055717826, AMSTERDAM, NL ISSN: 1078-1439, DOI: 10.1016/j.urolonc.2011.01.006
- DOLORES DI VIZIO ET AL: "An absence of stromal caveolin-1 is associated with advanced prostate cancer, metastatic disease spread and epithelial Akt activation", CELL CYCLE, vol. 8, no. 15, 1 August 2009 (2009-08-01) , pages 2420-2424, XP055070023, ISSN: 1538-4101, DOI: 10.4161/cc.8.15.9116
- VANVELDHUIZEN, P. J. ET AL.: 'Differential expression of neuropilin-1 in malignant and benign prostatic stromal tissue' ONCOLOGY REPORTS, [Online] vol. 10, no. 5, 01 September 2003, pages 1067 - 1071, XP055510115 Retrieved from the Internet: <URL:https://doi.org/10.3892/or.10.5.1067>
- KNIGHT, J. M. ET AL.: 'Comprehensive site-specific whole genome profiling of stromal and epithelial colonic gene signatures in human sigmoid colon and rectal tissue' PHYSIOLOGICAL GENOMICS, [Online] vol. 48, no. 9, 01 September 2016, pages 651 - 659, XP055510122 Retrieved from the Internet: <URL:doi: 10.1152/physiolgenomics.00023.2016>
- ERHO, N. ET AL.: 'Discovery and validation of a prostate cancer genomic classifier that predicts early metastasis following radical prostatectomy' PLOS ONE, [Online] vol. 8, no. 6, 24 June 2013, XP055371391 Retrieved from the Internet: <URL:https://doi.org/10.1371/journal.pone.0 066855>
- RODRIGUEZ-BERRIGUETE, G. ET AL.: 'Clinical significance of both tumor and stromal expression of components of the IL -1 and TNF-a signaling pathways in prostate cancer' CYTOKINE, [Online] vol. 64, no. 2, 21 September 2013, pages 555 - 563, XP055510224 Retrieved from the Internet: <URL:DOI: 10.1016/j.cyto.2013.09.003>
- HÄGGLÖF, C. ET AL.: 'The stroma-a key regulator in prostate function and malignancy' CANCERS, [Online] vol. 4, no. 2, 29 May 2012, ISSN 2072-6694 pages 531 - 548, XP055510227 ISSN: 2072-6694 Retrieved from the Internet: <URL:https://pdfs.semanticscholar.org/b289/ 8fb3da99ab3d22725884544c34dfee499435.pdf>

## Description

### TECHNICAL FIELD OF THE INVENTION

The invention relates to a method for predicting prostate cancer metastasis by evaluating the expression of 8 specific genes in primary tumors of prostate cancer.

### BACKGROUND OF THE INVENTION

Prostate cancer (PCa) is the second leading cause of death from cancer in men in Chile and the world. According to the National Cancer Institute of the United States, deaths increase at an annual rate of 5.4%. The reason for this alarming growth is the lack of tools that allow the oncologist to predict which patients will develop a metastatic disease in order to intervene in a timely and appropriate manner. Thus, new and more effective methods are urged to predict the degree of aggressiveness of PCa, and thus determine which patients really require more aggressive treatments aimed at preventing the development of metastasis.

Research on the stromal role in the progression of PCa arises due to this need. The cancer cells are not isolated from their environment, but interact strongly with the tumor micro-structure or stroma, that is to say, the cells of the organ of origin of the tumor, in this case the prostate. The inventors have observed that the cells that accompany the tumor cells in the primary tumor, in this case in the prostate gland, participate in the ability of these to develop metastases.

Based on these important observations, the inventors developed the method of the invention, which allows for the evaluation of the degree of aggressiveness of PCa, studying prostatic stromal cells, specifically the pattern of expression of 8 specific genes.

In the state of the art there is no document that anticipates the method of the invention, although there are some similar approaches to the problem. For example, US2011236903 A1 (MCCLELLAND MICHAEL et al, 2011-09-29), suggests, especially in its claim 17, a prognostic method for a subject diagnosed with prostate cancer, comprising: (A) providing a sample of prostate tissue of said subject, wherein said sample comprises stromal cells of the prostate; (B) measuring the expression levels of one or more genes in said stromal cells, wherein said one or more genes are the prostate cancer signature genes; (C) comparing said measured expression levels to reference the expression levels of said one or more genes, wherein said reference expression levels are determined in non-cancerous prostate tissue stromal cells; and (D) if said measured expression levels are not significantly higher or lower than the reference expression levels, identify said subject with relatively better prognosis than if said measured expression levels are significantly higher or lower than the reference expression levels, or if said measured expression levels are significantly higher or lower than the reference expression levels, identify said subject as having a relatively worse prognosis than if said measured expression levels are not significantly higher or lower than the reference expression levels... Where the "prostate cancer signature" genes are chosen from Tables 3 and 4, where Table 3 describes 339 genes and Table 4 describes 146 genes. Where none of these 485 genes studied in this document coincide with the 8 genes studied in the present invention.

Vanveldhuizen, P. J. et al. (Oncology reports, vol. 10, no. 5, 2003, 1067-1071) describe a differential expression of neuropilin-1 in malignant and benign prostatic stromal tissue, said expression being high in the surrounding stroma of invasive/metastatic carcinoma US2013/085080 relates to a method for assessing prostate cancer, said method comprising determining whether or not a mammal having prostate cancer comprises an elevated level of expression of a KHDRBS3, NRP1, COL10A1, C20orf102, SSTR1, RRM2, F5, HSPC150, CDC2, TOP2A, CDH10, SERPINI1, TDO2, GRIN3A, COL2A1 or PCDHB10 nucleic acid, or a polypeptide encoded by said nucleic acid, wherein the presence of said elevated level indicates that said mammal is susceptible to a poor outcome. We also came across the international publication WO2014052930 (A2) (MERCOLA DANIEL et al 2014-04-03) that points to biomarkers for the diagnosis and prognosis of prostate cancer. In this document the expression of genes in tumor and stromal cells is studied, however, they select as biomarker the expression of 7 genes in tumor cells. That is to say, Mercola does not anticipate the method of the invention, and in addition the genes studied do not coincide with the genes of the invention.

The publication by Klein et al (European Urology 66 (2014) 550-560) discloses an assay of 17 genes to predict aggressiveness of prostate cancer. Where the authors identified 17 genes that represent multiple biological pathways that discriminate the aggressiveness of PCa in the tissue of the biopsy, despite tumor heterogeneity, multifocality and limited sampling at the time of biopsy. The authors do not directly correlate the studies of these genes with the development of metastasis. The 17 genes studied by Klein do not coincide with the genes studied in the present invention.

Erho et al (PLoS ONE 8(6): e66855) discloses a method for predicting prostate cancer metastasis by studying 22 markers in cancer cells of the primary tumor.

In this way the invention differs from what is known until now, and becomes the first diagnostic method that allows the formation of PCa metastasis to be predicted, becoming an invaluable tool when defining the most appropriate treatment for a patient with prostate cancer. The invention is defined in the appended claims.

### DESCRIPTION OF THE FIGURES

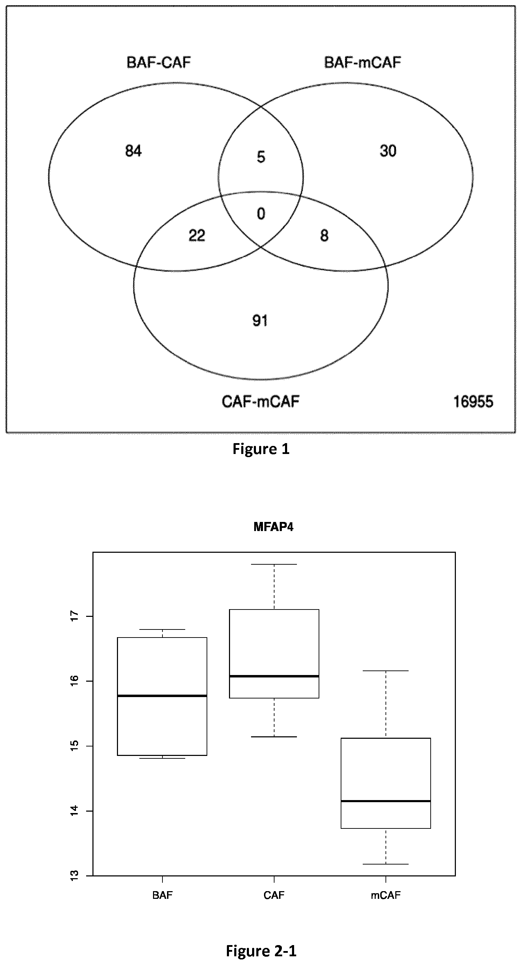

**Figure 1****.** Ven diagram of differential gene expression analysis in prostate stromal cells from patients without cancer (BAF), and in prostate cancer stroma of non-metastatic patients (CAF) and with metastases (mCAF). Each number indicates the number of genes differentially expressed between the groups compared. The number 8 means that there are 8 genes differentially expressed in mCAF compared to the other two groups and that they constitute the metastatic genetic signature of PCa.

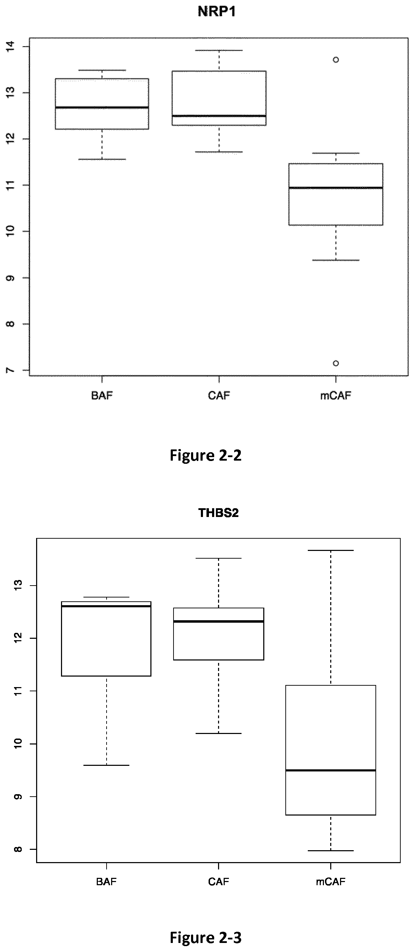

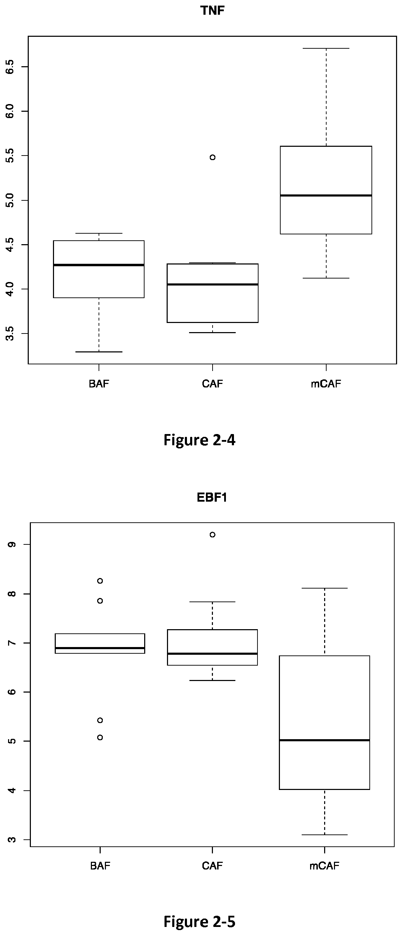

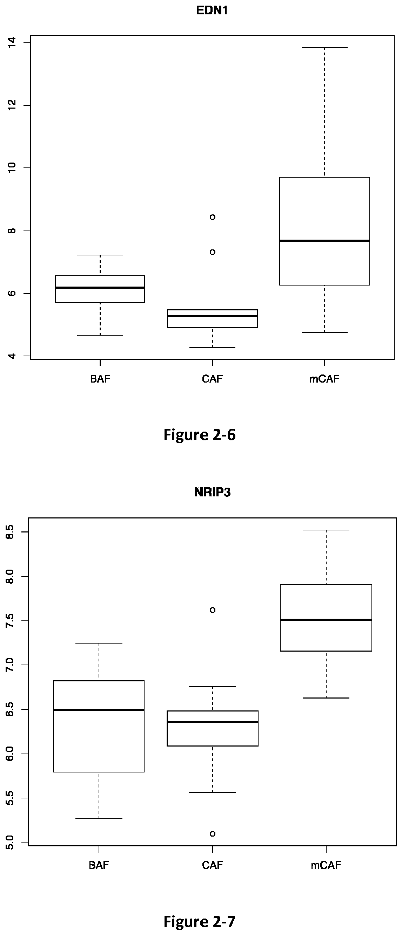

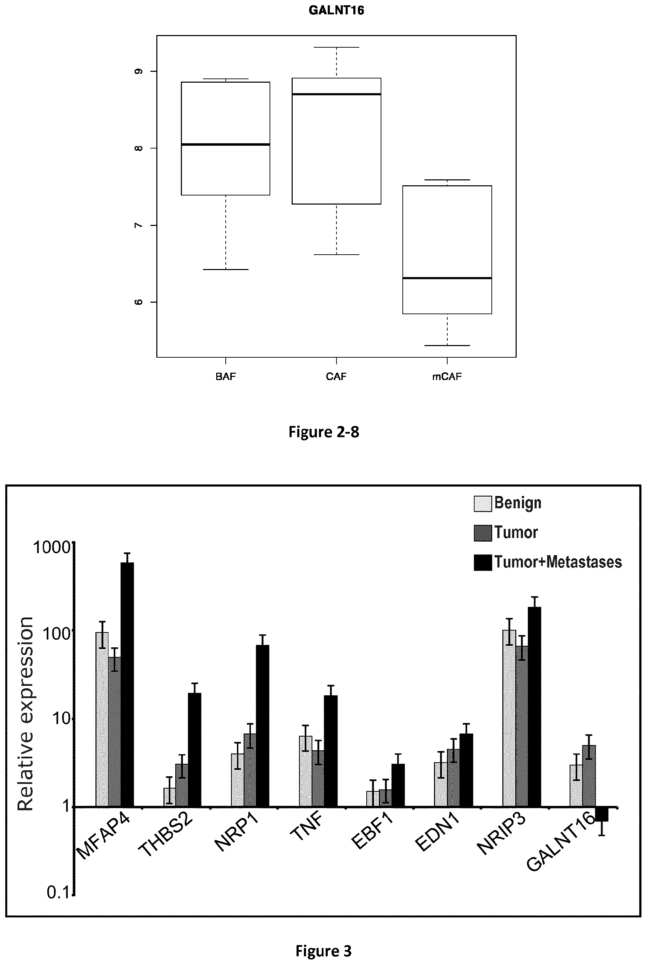

**Figure 2****:** Box plot diagrams that graphically show the dispersion of the expression of each gene in the samples of the 3 groups: BAF, CAF and mCAF. The expression was evaluated by studying the messenger RNAs in 10 samples of each group, using microarrays. The genes selected as genetic signature for the method of the invention showed the greatest significant difference between the three groups of samples and the smallest intra-group difference. Each box shows the intra-group dispersion, where the thick line is representative of the group mean, as seen in the graphs, the genes of the invention have differential expressions between the groups evaluated, and more especially between mCAF with respect to the CAF conditions and BAF. Using the statistical program "R", the dispersion of the expression values in all the samples was graphed:
   **Figure 2-1****:** Box plot graph of dispersion in expression values of the MFAP4 gene in prostate stromal cells from patients without cancer (BAF), with non-metastatic cancer (CAF) and with metastatic cancer (mCAF).
   **Figure 2-2****:** Box plot graph of dispersion in expression values of the NRP1 gene in prostate stromal cells from patients without cancer (BAF), with non-metastatic cancer (CAF) and with metastatic cancer (mCAF).
   **Figure 2-3****:** Box plot graph of dispersion in expression values of the THBS2 gene in prostate stromal cells from patients without cancer (BAF), with non-metastatic cancer (CAF) and with metastatic cancer (mCAF).
   **Figure 2-4****:** Box plot graph of dispersion in expression values of the TNF gene in prostate stromal cells from patients without cancer (BAF), with non-metastatic cancer (CAF) and with metastatic cancer (mCAF).
   **Figure 2-5****:** Box plot graph of dispersion in expression values of the EBF1 gene in prostate stromal cells from patients without cancer (BAF), with non-metastatic cancer (CAF) and with metastatic cancer (mCAF).
   **Figure 2-6****:** Box plot graph of dispersion in expression values of the EDN1 gene in prostate stromal cells from patients without cancer (BAF), with non-metastatic cancer (CAF) and with metastatic cancer (mCAF).
   **Figure 2-7****:** Box plot graph of dispersion in expression values of the NRIP3 gene in prostate stromal cells from patients without cancer (BAF), with non-metastatic cancer (CAF) and with metastatic cancer (mCAF).
   **Figure 2-8****:** Box plot graph of dispersion in expression values of the GALNT16 gene in prostate stromal cells from patients without cancer (BAF), with non-metastatic cancer (CAF) and with metastatic cancer (mCAF).
**Figure 3****:** Graph representing the relative expression from the data obtained by microarrays of the 8 genes that make up the genetic signature. The expression value of each gene in the 30 samples studied was averaged using the Agilent system. Samples obtained from metastatic PCa show an increase in the expression of the genes MFAP4, THSB2, NRP1, TNF, EBF1 and NRIP3, and a decrease in the expression of GALNT16 compared to benign samples or those with PCa without metastasis.

### DETAILED DESCRIPTION OF THE INVENTION

The invention describes a method of prognosis (used interchangeably in this description as "prediction") of metastasis in patients with prostate cancer, by studying the expression pattern of 8 specific genes: NRP-1, MFAP4, NRIP3, THBS2, TNF , EDN1, EBF1 and GALNT16, in PCa tumor stromal cells. Where, we conveniently study the expression of at least one of these 8 genes, or any of the possible combinations, of 2, 3, 4, 5, 6, 7 or the 8 genes identified by the inventors as markers of metastatic prostate cancer. Of these 8 biomarkers, the inventors have established that 7 of them have their expression increased in Metastatic Prostate Cancer, these are NRP-1, MFAP4, NRIP3, THBS2, TNF, EDN1, EBF1; whereas GALNT16 decreases its expression in this type of cancer. In both cases the increase or decrease of the expression is established by comparing the level of expression of the same genes with the normal expression control condition. The normal expression control condition is the tissue expression of prostatic stroma without cancer. In another embodiment, the expression control condition is the normal expression in prostatic stromal tissue with non-metastatic cancer.

To determine the genes of the genetic signature of the invention, the inventors studied the expression of all genes in three types of samples, firstly, prostate stromal cells from patients without cancer (used interchangeably in this description as "without neoplasia"), what is known in the art as BAF (benign tissue associated fibroblast), secondly, in prostatic stroma of patients with non-metastatic cancer what is known in the art as CAF (carcinoma associated fibroblast) and thirdly, in prostatic stroma of patients with metastatic cancer, which is known in the art as mCAF (metastatic carcinoma associated fibroblast). Of the 30,000 genes studied, there were 16,955 that were expressed, and of these, the inventors were able to select 8 genes that have a differentiated expression in mCAF with respect to CAF and BAF, those which constitute the genetic signature of the present invention. The greater the dispersion between the gene expression data, the lower the statistical value of the result for a given gene, so the inventors discarded all those genes with a large dispersion in their expression in a given condition (BAF CAF or mCAF) and those with a similar expression pattern (low dispersion) were left. On the other hand, greater distance between the different groups on the vertical axis means that the expression values for that gene are statistically different in the groups compared. Therefore, the inventors selected as a second condition that there was, in addition to a low intra-group dispersion, a high inter-group differentiation. Therefore, it can be seen that mCAF samples have a dispersion by quantiles different from BAF or CAF, these last two present a similar dispersion. These results are shown in Figure 2, where Figures 2-1 to 2-8 show the result for each of the genes of the invention.

As can be seen in figure 2, the expression differences between the mCAF group and CAF and BAF are clear in each of the genes studied, so that for the embodiment of the invention the expression of all the genes of the invention can be studied or of only one or all of the possible combinations, since one positive result according to the method of the invention is sufficient to predict metastatic prostate cancer, where additional results validate and reaffirm the prognosis of metastasis in a patient.

To develop the method of the present invention it is necessary first to obtain a sample of prostatic stroma. In an embodiment of the invention these samples are obtained from biopsies that are performed on patients with suspected cancer or already diagnosed with PCa, so that they would not mean an additional or different procedure from those usually performed for these patients. In another embodiment of the invention, a sample of prostatic stroma is obtained in a procedure additional to the biopsy that is performed on a patient with suspected cancer or already diagnosed with PCa. Although the method of the invention studies the expression of genes in the stroma, the analyses can be performed on complex samples with different cell types, containing stromal cells.

Thus, a preferred embodiment of the present invention comprises obtaining mRNA tissue from PCa primary tumors, and amplifying by RT PCR the genes chosen from NRP-1, MFAP4, NRIP3, THBS2, TNF, EDN1, EBF1 and GALNT16, and establishing the expression pattern of said genes. Where, if an overexpression of genes NRP-1, MFAP4, NRIP3, THBS2, TNF, EDN1, EBF1, or a decrease in the expression of GALNT16 is observed, this result is indicative of metastasis in patients with prostate cancer.

To establish the expression pattern of these genes, any method available in the art can be used at the time of performing the invention. If the gene product is RNA, it can be evaluated by microarray, SAGE, western blotting, RT-PCR, TRAC, quantitative PCR, multiplex qPCR or qNPA, or any other technique available at the time of performing the invention.

In a preferred embodiment the expression of the genes of the invention is established by Real Time PCR on the mRNA of the tissue sample.

In another preferred embodiment the expression of the genes of the invention is established by microarray on the mRNA of the tissue sample.

In another preferred embodiment the expression of the genes of the invention is established by multiplex qPCR on the mRNA of the tissue sample.

In another embodiment of the invention the level of expression of the genes is determined by studying the proteins encoded by the genes NRP-1, MFAP4, NRIP3, THBS2, TNF, EDN1, EBF1 and GALNT16. Where the concentration of these proteins in the sample can be evaluated by ELISA, mass spectrometry, proteomic techniques, or immunohistochemistry, or any other technique available at the time of carrying out the invention. In each case the expression of the gene product, in this case proteins, of at least one of the genes NRP-1, MFAP4, NRIP3, THBS2, TNF, EDN1, EBF1 and GALNT16 should be compared with normal expression conditions, for example from patients without neoplasia (BAF) and establishing if there is an overexpression of the genes NRP-1, MFAP4, NRIP3, THBS2, TNF, EDN1, EBF1 or a silencing or diminution of expression of the GALNT16 gene. Where this difference for any of the genes or preferably all of them, including all possible combinations are indicative of the development of a metastatic disease.

In second place, the invention also describes a kit for predicting metastasis in prostate cancer by the method of the invention, where this kit consists in means for quantifying the expression products of the genes NRP-1, MFAP4, NRIP3, THBS2, TNF, EDN1, EBF1 and GALNT16, in a sample comprising prostatic tissue, wherein the means provided in the kit comprise reagents, solutions and physical support elements, wherein the reagents correspond to a polypeptide or oligonucleotide for detecting the level of expression of genes which reagents correspond to either: specific antibodies for binding to proteins corresponding to any of the products of the expression of the group of genes consisting of NRP-1. MFAP4, NRIP3, THBS2, TNF, EDN1, EBF1 and GALNT16 genes; and/or Z specific oligonucleotides , primers or probes, to hybridize to any of the products of the expression of the group of genes consisting of NRP-1, MFAP4, NRIP3, THBS2, TNF, EDN1, EBF1 and GALNT16. The solutions of the kit include the necessary solutions to determine the level of expression of the genes in suitable equipment. On the other hand, the means of physical support can be containers or tubes to contain the different reagents and solutions of the kit.

It is evident to the person skilled in the art that there are numerous PCR primers or probes that can be designed to perform these amplifications, and that the primers or probes used are not limiting for the embodiment of the invention. Both primers or probes described in the literature for these genes may be used or new primers or probes designed for this purpose, where all of these embodiments are within the scope of the present invention.

In third place, the invention also relates to the use of the kit described above or of a kit comprising means to quantify the products of genes NRP-1, MFAP4, NRIP3, THBS2, TNF, EDN1, EBF1 and GALNT16, in a sample that comprises prostatic stromal cells for the prediction of metastasis in prostate cancer, said use comprising establishing whether the expression of the genes NRP-1, MFAP4, NRIP3, THBS2, TNF, EDN1, EBF1 is increased or if the expression of the GALNT16 gene is diminished, which correlates with the prognosis of metastasis in prostate cancer.

### EXAMPLES

### Example 1. Test to establish the biomarkers.

Prostate stromal cells were obtained from patients without evidence of neoplasia (BAF), from PCa primary tumors from patients who had developed clinical metastases (mCAF) and from patients with PCa who had not developed clinical metastasis (CAF). All these samples were obtained from puncture biopsies by means of tissue explants with the respective informed consent from the donors. Thirty samples were collected, 10 for each group.

The cells obtained were cultured in the laboratory until a critical number of them were obtained to carry out the studies.

To perform the gene expression experiments, mRNA was first extracted from each of the 30 samples (10 for each condition) with the Trizol reagent (Invitrogen) following the manufacturer's instructions. To determine the integrity of the extracted total RNA, a sample of it was subjected to electrophoresis in a 1% agarose gel stained with ethidium bromide. The RNA was quantified at 260 nm in a Nanodrop 1000 (Thermo).

Each sample was evaluated by a direct hybridization assay of only one color, using the Agilent Human Gene expression 4x44Kv2v system and runs in the iScan system. The gene expression data were analyzed by means of bioinformatics with the statistical program "R".

Gene expression profiles were made by microarrays of these stromal cells for 30,000 genes in each case. The results indicate that the prostatic stroma in patients with metastatic PCa present gene expression profiles different from the prostatic stroma of patients without cancer or with non-metastatic PCa. From the 30,000 genes studied, 16,955 genes were found that were expressed in all groups; from these genes, a series of genes whose differential expression had a high degree of significance was first selected.

The results demonstrate that mCAFs stromal cell samples exhibit a differential gene expression profile, as compared to stromal samples of BAFs or CAFs. Among the differentially expressed genes, we established 8 that presented the greatest statistical difference between samples of patients with metastatic PCa versus benign samples or with PCa without metastasis. These results are schematized in Figure 1. These 8 genes are: NRP-1, MFAP4, NRIP3, THBS2, TNF, EDN1, EBF1 and GALNT16. The relative differential expression for each of these genes in the different groups studied is schematized in Figure 2, where there is a box plot for each of these 8 genes. Additionally, all this data is summarized in Figure 3.

Example 2. Study of expression of the genes of the invention in patients with PCa.

Primers for PCR (polymerase chain reaction) were designed for the genes identified in example 1, and 50 cases of patients without evidence of neoplasia (BAF), with PCa and clinical metastasis (mCAF) and with PCa without clinical metastasis were studied (CAF), through real-time PCR. The used primers are shown in Table 1.

In each case, a puncture biopsy was taken by means of tissue explants, from which a sample of total mRNA was obtained, which includes RNA from stromal cells and from tumor cells. Total RNA extraction was performed by a commercial kit (Qiagen) following the manufacturer's instructions. To determine the integrity of the extracted total RNA, a sample of it was subjected to electrophoresis in a 1% agarose gel stained with ethidium bromide. The RNA was quantified at 260 nm in a Nanodrop 1000 (Thermo).

As positive control of the reaction the 18S gene was quantified and to normalize the results based on the total amount of stroma of the sample, the messenger of Vimentina was quantified, the primers used in each case are also included in Table 1.

**Table 1: Primers used for the genes of the invention.**

| **Gene** | **Direct primers (5'-3')** | **Reverse primers (5'-3')** | **Product** |
|---|---|---|---|
| THBS2 | CGTGGACAATGACCTTGTTG | GCCATCGTTGTCATCATCAG | 169 pb |
| MFAP4 | TATCTACGCCCAGGGCTATC | CAAAGCCCAGCTTGTAGTCG | 182 pb |
| NRP1 | AAATGCGAATGGCTGATTCA G | CTCCATCGAAGACTTCCACG TAGT | 121 pb |
| TNF | ATCTACCTGGGAGGCGTCTT | GAGTGGCACAAGGAACTGGT | 99 pb |
| EBF1 | GCATCCAACGGAGTGGAAG | GATTTCCGCAGGTTAGAAGG C | 175 pb |
| EDN1 | CGTAGAAGTCTGGTCTAATG TGTCA | GTACATCAAAATTCAATCCA ACCTC | 250 pb |
| NRIP3 | ATTTCCATTGATGACAAGCT TCC | CTGCTTTTAACTCTGGTAAA GTGGA | 155 pb |
| GALNT1 6 | AACCTCTGCTCTGGATTGAA | AGACACTTGGCTGCTGAC | 127 pb |
| Vimentina | AAAGTGTGGCTGCCAAGAAC | AGCCTCAGAGAGGTCAGCA A | 74 pb |
| 18S | GTAACCCGTTGAACCCCATT | CCATCCAATCGGTAGTAGCG | 151 pb |

The obtained data corroborates that which was established in the microarrays of example 1, finding that when comparing the relative expression of these gene products in all cases there is a correlation between the overexpression of the NRP-1, MFAP4, NRIP3, THBS2, TNF, EDN1 and EBF1, and the decreased expression or silencing of GALNT16 and the clinical manifestations of these patients. That is to say, in the cases of patients with metastasis (mCAF) an increase in mRNA expression of NRP-1, MFAP4, NRIP3, THBS2, TNF, EDN1 and EBF1 is seen, and the decrease in the expression of GALNT16 mRNA, with respect to the expression in samples of patients without neoplasia (BAF) and with respect to the expression in samples of patients with non-metastatic prostate cancer.

From this result it can be determined that it is possible to clearly identify, by means of gene expression of stromal cells of the primary tumor, patients with metastatic prostate cancer.

It will be apparent to the person skilled in the art that it is possible to develop a kit for the method of the invention, including for example the PCR primers indicated in this example and the reagents needed to perform a real-time PCR, or any other mRNA quantitative technique or another product of expression of the genes of the invention. Likewise, it is possible to develop microarrays that determine the level of expression of these genes. Given that the genes are known and that the techniques for designing primers or probes are standardized in the art, the method of the invention can be performed with any pair of primers or probes that specifically amplify these genes, this not being a limitation of the method.

## Claims

1. Ex vivo method for the prognosis of metastasis in prostate cancer, **CHARACTERIZED in that** it comprises the following steps:
a) determining the level of expression of the following genes: NRP-1, MFAP4, NRIP3, THBS2, TNF, EDN1, EBF1 and GALNT16, in a biological sample containing stromal cells of a prostate cancer primary tumor;
b) establishing whether the expression of the genes NRP-1, MFAP4, NRIP3, THBS2, TNF, EDN1, EBF1 is increased or if the expression of the GALNT16 gene is diminished, which correlates with the prognosis of metastasis in prostate cancer.

2. Method according to claim 1, **CHARACTERIZED in that** the level of expression of these genes is established by comparing the expression of each gene product evaluated against the normal expression control condition.

3. Method according to claim 2, **CHARACTERIZED in that** the normal expression condition is normal expression in prostatic stromal tissue.

4. Method according to claim 3, **CHARACTERIZED in that** the relative level of RNA transcripts of the genes NRP-1, MFAP4, NRIP3, THBS2, TNF, EDN1, EBF1 and GALNT16 is determined.

5. Method according to claim 2, **CHARACTERIZED in that** the level of RNA expression is determined by microarray, SAGE, western blotting, RT-PCR, TRAC, quantitative PCR, multiplex qPCR or qNPA.

6. Method according to claim 3, **CHARACTERIZED in that** the level of proteins encoded in the genes NRP-1, MFAP4, NRIP3, THBS2, TNF, EDN1, EBF1 and GALNT16 is determined.

7. Method according to claim 4, **CHARACTERIZED in that** the level of expression of proteins is determined by ELISA, mass spectrometry, proteomic techniques, or immunohistochemistry.

8. Method according to claim 1, **CHARACTERIZED in that** the biological sample containing stromal cells from a prostate cancer primary tumor was obtained by a biopsy of a prostate cancer primary tumor.

9. Kit to predict metastasis in prostate cancer according to claim 1, **CHARACTERIZED in that** it consists in means to quantify the products of the group of genes consisting of NRP-1, MFAP4, NRIP3, THBS2, TNF, EDN1, EBF1 and GALNT16, in a sample that comprises prostatic stromal cells, wherein the means to quantify comprises reagents, solutions and physical support elements, wherein the reagents correspond to a polypeptide or an oligonucleotide to detect level of expression of the genes, which reagents correspond to:
- specific antibodies for binding to proteins corresponding to the products of the expression of the group of genes consisting of NRP-1, MFAP4, NRIP3, THBS2, TNF, EDN1, EBF1 and GALNT16, and/or
- specific oligonucleotide primers or probes to hybridize to the products of the expression of the group of genes consisting of NRP-1, MFAP4, NRIP3, THBS2, TNF, EDN1, EBF1 and GALNT16.

10. Use of the kit according to claim 9 to quantify the products of genes NRP-1, MFAP4, NRIP3, THBS2, TNF, EDN1, EBF1 and GALNT16, in a sample that comprises prostatic stromal cells for the prediction of metastasis in prostate cancer, said use comprising establishing whether the expression of the genes NRP-1, MFAP4, NRIP3, THBS2, TNF, EDN1, EBF1 is increased or if the expression of the GALNT16 gene is diminished, which correlates with the prognosis of metastasis in prostate cancer.

## Patentansprüche

1. Ex-Vivo-Verfahren zur Prognose von Metastasen bei Prostatakrebs, **DADURCH GEKENNZEICHNET, dass** es die folgenden Schritte umfasst:
a) Bestimmung des Expressionsgrads der folgenden Gene: NRP-1, MFAP4, NRIP3, THBS2, TNF, EDN1, EBF1 und GALNT16 in einer biologischen Probe, die Stromazellen eines Prostatakrebstumors enthält;
b) Feststellung, ob die Expression der Gene NRP-1, MFAP4, NRIP3, THBS2, TNF, EDN1, EBF1 erhöht ist oder ob die Expression des GALNT16-Gens vermindert ist, was mit der Prognose einer Metastasierung bei Prostatakrebs korreliert.

2. Verfahren nach Anspruch 1, **DADURCH GEKENNZEICHNET, dass** der Expressionsgrad dieser Gene durch Vergleich der Expression jedes bewerteten Genprodukts mit der normalen Expressionskontrollbedingung festgestellt wird.

3. Verfahren nach Anspruch 2, **DADURCH GEKENNZEICHNET, dass** die normale Expressionsbedingung eine normale Expression in Prostata-Stromagewebe ist.

4. Verfahren nach Anspruch 3, **DADURCH GEKENNZEICHNET, dass** die relative Menge an RNA-Transkripten der Gene NRP-1, MFAP4, NRIP3, THBS2, TNF, EDN1, EBF1 und GALNT16 bestimmt wird.

5. Verfahren nach Anspruch 2, **DADURCH GEKENNZEICHNET, dass** die Menge der RNA-Expression durch Microarray, SAGE, Western Blotting, RT-PCR, TRAC, quantitative PCR, Multiplex-qPCR oder qNPA bestimmt wird.

6. Verfahren nach Anspruch 3, **DADURCH GEKENNZEICHNET, dass** die Menge an Proteinen, die in den Genen NRP-1, MFAP4, NRIP3, THBS2, TNF, EDN1, EBF1 und GALNT16 kodiert werden, bestimmt wird.

7. Verfahren nach Anspruch 4, **DADURCH GEKENNZEICHNET, dass** der Expressionsgrad der Proteine durch ELISA, Massenspektrometrie, proteomische Techniken oder Immunhistochemie bestimmt wird.

8. Verfahren nach Anspruch 1, **DADURCH GEKENNZEICHNET, dass** die biologische Probe, die Stromazellen von einem Prostatakrebs-Primärtumor enthält, durch eine Biopsie eines Prostatakrebs-Primärtumors erhalten wurde.

9. Kit zur Vorhersage von Metastasen bei Prostatakrebs nach Anspruch 1, **DADURCH GEKENNZEICHNET, dass** er aus Mitteln zur Quantifizierung der Produkte der Gruppe von Genen besteht, die wiederum aus NRP-1, MFAP4, NRIP3, THBS2, TNF, EDN1, EBF1 und GALNT16 bestehen, in einer Probe, die Prostata-Stromazellen umfasst, wobei das Mittel zum Quantifizieren Reagenzien, Lösungen und physikalische Trägerelemente umfasst und wobei die Reagenzien einem Polypeptid oder einem Oligonukleotid entsprechen, um den Expressionsgrad der Gene nachzuweisen, wobei die Reagenzien Folgendem entsprechen:
- spezifischen Antikörpern zur Bindung an Proteine, die den Expressionsprodukten der Gruppe von Genen entsprechen, die aus NRP-1, MFAP4, NRIP3, THBS2, TNF, EDN1, EBF1 und GALNT16 bestehen, und/oder
- spezifischen Oligonukleotid-Primern oder -Sonden zur Hybridisierung an den Expressionsprodukten der Gruppe von Genen bestehend aus NRP-1, MFAP4, NRIP3, THBS2, TNF, EDN1, EBF1 und GALNT16.

10. Verwendung des Kits nach Anspruch 9 zur Quantifizierung der Produkte der Gene NRP-1, MFAP4, 10 NRIP3, THBS2, TNF, EDN1, EBF1 und GALNT16 in einer Probe, die Prostatastromazellen umfasst, für die Vorhersage von Metastasen bei Prostatakrebs, wobei die Verwendung die Feststellung umfasst, ob die Expression der Gene NRP-1, MFAP4, NRIP3, THBS2, TNF, EDN1, EBF1 erhöht ist oder ob die Expression des GALNT16-Gens verringert ist, was mit der Prognose von Metastasen bei Prostatakrebs korreliert.

## Revendications

1. Méthode ex vivo de pronostic de métastases dans le cancer de la prostate, **CARACTÉRISÉ en ce qu'**il comprend les étapes suivantes :
a) détermination du niveau d'expression des gènes suivants : NRP-1, MFAP4, NRIP3, THBS2, TNF, EDN1, EBF1 et GALNT16, dans un échantillon biologique contenant des cellules stromales d'une tumeur primitive du cancer de la prostate;
b) établir si l'expression des gènes NRP-1, MFAP4, NRIP3, THBS2, TNF, EDN1, EBF1 est augmentée ou si l'expression du gène GALNT16 est diminuée, ce qui est en corrélation avec le pronostic des métastases dans le cancer de la prostate.

2. Méthode selon la revendication 1, **CARACTÉRISÉ en ce que** le niveau d'expression de ces gènes est établi en comparant l'expression de chaque produit génique évalué avec la condition de contrôle d'expression normale.

3. Méthode selon la revendication 2, **CARACTÉRISÉ en ce que** la condition d'expression normale est une expression normale dans le tissu stromal prostatique.

4. Méthode selon la revendication 3, **CARACTÉRISÉ en ce que** le niveau relatif de transcrits d'ARN des gènes NRP-1, MFAP4, NRIP3, THBS2, TNF, EDN1, EBF1 et GALNT16 est déterminé.

5. Méthode selon la revendication 2, **CARACTÉRISÉ en ce que** le niveau d'expression d'ARN est déterminé par microréseau, SAUGE, western blot, RT-PCR, trac, PCR quantitative, multiplex qPCR ou qNPA.

6. Méthode selon la revendication 3, **CARACTÉRISÉ en ce que** le niveau de protéines codées dans les gènes NRP-1, MFAP4, NRIP3, THBS2, TNF, EDN1, EBF1 et GALNT16 est déterminé.

7. Méthode selon la revendication 4, **CARACTÉRISÉ en ce que** le niveau d'expression de protéines est déterminé par ELISA, spectrométrie de masse, techniques protéomiques ou immunohistochimie.

8. Méthode selon la revendication 1, **CARACTÉRISÉ en ce que** l'échantillon biologique contenant des cellules stromales d'une tumeur primaire de cancer de la prostate a été obtenu par une biopsie d'une tumeur primaire de cancer de la prostate.

9. Kit de prédiction de métastases dans le cancer de la prostate selon la revendication 1, **CARACTÉRISÉ en ce qu'**il comporte des moyens pour quantifier les produits du groupe de gènes constitué de NRP-1, MFAP4, NRIP3, THBS2, TNF, EDN1, EBF1 et GALNT16, dans un échantillon qui comprend des cellules stromales prostatiques, dans lequel les moyens de quantification comprennent des réactifs, des solutions et des éléments de support physique, dans lequel les réactifs correspondent à un polypeptide ou à un oligonucléotide pour détecter le niveau d'expression des gènes, lesquels réactifs correspondent à :
- des anticorps spécifiques de liaison aux protéines correspondent aux produits d'expression du groupe de gènes constitué par NRP-1, MFAP4, NRIP3, THBS2, TNF, EDN1, EBF1 et GALNT16, et/ou
- des amorces ou sondes oligonucléotidiques spécifiques pour s'hybrider aux produits de l'expression du groupe de gènes constitué de NRP-1, MFAP4, NRIP3, THBS2, TNF, EDN1, EBF1 et GALNT16.

10. Utilisation du kit selon la revendication 9 pour quantifier les produits des gènes NRP-1, MFAP4, 10 NRIP3, THBS2, TNF, EDN1, EBF1 et GALNT16, dans un échantillon qui comprend des cellules stromales prostatiques pour la prédiction de métastases dans le cancer de la prostate, ladite utilisation comprenant l'établissement si l'expression des gènes NRP-1, MFAP4, NRIP3, THBS2, TNF, EDN1, EBF1 est augmentée ou si l'expression du gène GALNT16 est diminuée, ce qui est en corrélation avec le pronostic de métastases dans le cancer de la prostate.
